# EUROPÄISCHE PATENTANMELDUNG

(11) **EP 0 967 242 A2**
(43) Veröffentlichungstag der Anmeldung: **29.12.1999**
(21) Anmeldenummer: 99108833.7
(22) Anmeldetag: 04.05.1999
(51) Int. Cl.: C08J 7/00, A61L 15/00, A61L 17/00, A61L 25/00, A61L 29/00, A61L 31/00

(54) **Bioaktive Polymere mit fixierten Immunoadjuvantien**

(30) Priorität: 26.05.1998 DE 19823391
(71) Anmelder: Creavis Gesellschaft für Technologie und Innovation mbH, 45764 Marl (DE)
(72) Erfinder: Kunz, Roland, 45770 Marl (DE); Schindler, Fritz, Dr., 45879 Gelsenkirchen (DE)

(57) **Zusammenfassung**

Die Erfindung betrifft Polymere, die auf ihrer Oberfläche fixierte Immunuadjuvantien tragen. Das Basispolymer und das Immunoadjuvans können durch ionische oder kovalente Bindungen miteinander verknüpft sein, gegebenenfalls über Brückenmoleküle. Die Polymere eignen sich zur Herstellung von Erzeugnissen, die frei oder möglichst frei von Bakterien oder anderen schädlichen Mikroorganismen sein und bleiben sollen oder mit Tumorzellen in Berührung kommen.

## Beschreibung

Die Erfindung betrifft neue bioaktive Polymere, die auf ihrer Oberfläche fixierte Immunoadjuvantien tragen. Die Erfindung betrifft weiterhin ein Verfahren zur Herstellung solcher Polymere sowie deren Verwendung in der Tumortherapie und zur Herstellung von medizin- oder veterinärmedizintechnischen Erzeugnissen, die beim Einsatz im menschlichen oder tierischen Körper frei von Mikroorganismen bleiben sollen.

### 1. Stand der Technik

Die Erfindung liegt primär auf dem Gebiet der unspezifischen zellulären Immunantwort. Es ist seit längerem bekannt, daß bestimmte Stoffe - sogenannte Immunoadjuvantien - die zelluläre unspezifische Immunantwort stimulieren. Hierunter versteht man, daß Freßzellen, wie Makrophagen, dargebotene Immunoadjuvantien aufnehmen, z.B. durch Phagocytose, und dann zersetzen, wobei Signalstoffe ausgeschieden werden, die sowohl die Aktivität vorhandener als auch die Bildungs- und Reifungsgeschwindigkeit neu entstehender Freßzellen fördern. Ergebnis dieses Prozesses ist die Stimulierung des unspezifischen, also gegen unterschiedliche Fremdstoffe gerichteten Immunsystems. Da Immunoadjuvantien zersetzende Freßzellen über Signalstoffe und Kontaktmechanismen auch mit dem B- und dem T-Lymphozytensystem interagieren, wird über Immunoadjuvantien sekundär auch das spezifische Immunsystem aktiviert. Hier ist die Forschung noch nicht abgeschlossen; so lassen D.Luzio et al. ( Int.J.Cancer, **24**, 773-779, 1979) offen, inwieweit die Wirkung des Immunoadjuvans Glucan, ein Polysaccharid aus Hefe, Makrophagen- oder Lymphozyten-bedingt ist.

Tumorzellen werden vom Immunsystem als an sich körperfremd erkannt und nach Aktivierung des-Immunsystems ebenfalls verstärkt attackiert (D.Luzio et al, a.a.O.; Immun. **57**, No. 8, 2495ff., 1978).

Beispiele für bekannte Immunoadjuvantien sind:
- Freund's Complete Ajuvant: hitzegetötete Mycobakterien in Mineralöl (Adv.Tuberc.Res, **1956**, 7, 130-148)
- Trehalosedimycolsäure; ein Bestandteil von Freund's Complete Adjuvans (Infect.Immun. **57**, No.8, Seiten 2490 ff (1987)
- Monophosphoryllipid A: ein nicht toxisches Derivat von Diphosphoryllipid aus Salmonella-Bakterien (Rev.Infect.Dis. **6**, 567-572, 1983)
- Chitin: β-(1,4)-Poly-N-acetyl-D-glucosamin (Vaccine, **2**, 93-99, 1983)
- Glucan: Polysaccharid aus Hefe (J.Leucocyte Biol. **42**, 95-105, 1987)
- Biostim: Extrakt aus Zellen von Klebsiella pneumoniae (Current Therapeutic Research **57**, 16-26, 1996)
- N-Acetylmuramyldipeptid: Isolat aus der Zellwand von Bakterien (Biochem.Biophys.Res.Comm. **59**, 1317-1325, 1974)

Neben diesen natürlichen Immunoadjuvantien, die sich aus biologischem Material gewinnen lassen, gibt es auch synthetische Adjuvantien. Beispiele hierfür sind:
- Synthetische Glycolipide (Advances in the Biosciences **68**, 429ff., (1988)
- Trehalosediester (Medicinal Res.Rev.**6**, No.3,243-274 1986)

Die Wirkung von Immunoadjuvantien läßt sich in ihrer Dauer und Intensität erhöhen, wenn man sie mit Hilfsstoffen kombiniert. Diese Hilfsstoffe präsentieren dem Immunsystem das Adjuvans in einer besonders stimulierenden Form bzw. bewirken die Anlage eines Adjuvans-Depots. So werden bei Freund's Adjuvans die abgetöteten Mycobakterien in einer Mineralölemulsion dargeboten, was zu einer besonders aktiven und lange anhaltenden Stimulierung des Immunsystems führt. Die in der Emulsion enthaltenen abgetöteten Bakterien bzw. Bestandteile von ihnen gelangen nach und nach an die Oberfläche und werden den Freßzellen präsentiert. Einen dem Mineralöl entsprechenden Effekt zeigen auch andere Öle, an die, z.T. in Kombination mit Tensiden. Adjuvantien kovalent oder nicht kovalent gebunden sind. Beispiele hierzu finden sich in US 4 772 466, EP 324 455 und US 5 026 543. In ähnlicher Weise wie Öle wirkt auch Aluminiumhydroxid. Die Adjuvantien werden daran adsorbiert und nach und nach abgegeben. Charakteristisch für die Kombinationen aus Adjuvans und Hilfsstoff ist, daß sie im Körper mobil sind und beispielsweise mit dem Blutstrom transportiert werden.

### 2. Kurzbeschreibung der Erfindung

Ein Gegenstand der Erfindung sind neue Polymere, die auf ihrer Oberfläche fixierte Adjuvantien tragen. Bevorzugt sind die Immunoadjuvantien durch ionische oder kovalente Bindungen auf der Polymeroberfläche fixiert.

Ein anderer Gegenstand der Erfindung ist ein Verfahren zur Herstellung der bevorzugten neuen Polymeren, bei dem man ein Basispolymer und ein Immunoadjuvans über geeignete funktionelle Gruppen ionisch oder kovalent miteinander verknüpft. Die Verknüpfung kann unmittelbar oder mittelbar über ein Brückenglied erfolgen.

Ein weiterer Gegenstand der Erfindung ist ein Verfahren zur Herstellung von Erzeugnissen oder Teilen von Erzeugnissen, bei deren bestimmungsgemäßer Verwendung es erforderlich oder wünschenswert ist, daß sie frei oder möglichst frei von schädlichen Mikroorganismen sind. Das Verfahren ist dadurch gekennzeichnet, daß man dabei die erfindungsgemäßen Polymeren verwendet.

Ein anderer Gegenstand der Erfindung ist ein Verfahren zur Herstellung von Erzeugnissen oder Teilen von Erzeugnissen, die bei bestimmungsgemäßer Verwendung mit Tumorzellen in Berührung kommen. Dieses Verfahren ist ebenfalls dadurch gekennzeichnet, daß man dabei die erfindungsgemäßen Polymere verwendet.

Schließlich sind auch die zuvor genannten Erzeugnisse oder Teile von Erzeugnissen ein Gegenstand der Erfindung.

### 3. Vorteile der Erfindung

Durch die Fixierung der Immunoadjuvantien werden hauptsächlich zwei Effekte erzielt. Zum einen wird die Mobilität der Immunoadjuvantien aufgehoben, wodurch ihre Wirkung auf die sie tragende Polymeroberfläche und ihre Umgebung konzentriert wird. Zum anderen fördert die Fixierung die Wirkung der Immunoadjuvantien ähnlich wie der Einsatz von Hilfsstoffen. Dies liegt offenbar daran, daß die Freßzellen die Immunoadjuvantien infolge ihrer Fixierung auf der Polymeroberfläche nicht ohne weiteres umschließen und lysieren können, vielmehr zu einer langanhaltenden, intensiven und die Aktivität steigernden Beschäftigung mit dem Adjuvans gezwungen sind. Die so aktivierten Freßzellen verhindern in ihren Nachbarschaft die Besiedelung der Polymeroberfläche mit Bakterien oder anderen schädlichen Mikroorganismen bzw. weiden bereits auf der Oberfläche siedelnde Bakterien ab. Liegt das erfindungsgemäße Polymer, z.B. in Form einer Folie, im Kontakt mit bzw. in räumlicher Nähe zu einem Tumor vor, so werden auch die Tumorzellen primär von den aktivierten Freßzellen angegriffen und sekundär, nach Aktivierung der T- und B-Lymphozyten, auch vom spezifischen Immunsystem bekämpft.

### 4. Beschreibung der Erfindung

### 4.1 Geeignete Basispolymere

Als Basispolymere werden Polymere bezeichnet, die erfindungsgemäß die Immunoadjuvantien tragen. Geeignete Basispolymere sind vor allem Polymere mit funktionellen Gruppen, welche eine ionische oder kovalente Bindung zu den Adjuvantien auszubilden vermögen.

Geeignete funktionelle Gruppen sind beispielsweise Hydroperoxygruppen. Hydroxylgruppen, Carboxylgruppen und Aminogruppen. Sie können zum Teil über Monomere bei der Herstellung der Polymeren eingebracht oder durch Wahl geeigneter Mengenverhältnisse der zur Herstellung verwendeten Komponenten erzeugt werden. In beiden Fällen sind die funktionellen Gruppen im Bulk vorhanden. Geeignete Monomere sind z.B. 2-Hydroxyethyl(meth)acrylat und 4-Hydroxybutyl(meth)acrylat, die Hydroxylgruppen liefern. Saure oder potentiell saure Monomere, wie Acrylsäure, Maleinsäure, Acrylester oder Maleinsäureester oder Maleinsäureanhydrid, ergeben, gegebenenfalls nach Verseifung oder Hydrolyse, freie Carboxylgruppen. Carboxylgruppen sind auch vorhanden, wenn man bei der Polykondensation von Dicarbonsäuren mit Alkylendiaminen zu Polyamiden die ersteren im Überschuß einsetzt; im umgekehrten Fall erhält man Polyamide mit freien Aminogruppen. Letztere können auch in Polyurethanen durch Hydrolyse von überschüssigen Isocyanatgruppen entstehen. Alkyd- oder Melaminharze können, je nach den Mengenverhältnissen der Komponenten, freie Hydroxyl-, Carboxyl- oder Aminogruppen aufweisen.

Alternativ können funktionelle Gruppen nachträglich durch eine entsprechende Oberflächenbehandlung von nicht funktionalisierten Standardpolymeren erzeugt werden. Sauerstoffhaltige funktionelle Gruppen können z.B. durch UV-Strahlung, Hochfrequenz oder Mikrowellenplasma, Koronabehandlung, Elektronen- oder gamma-Strahlen, Beflammen der Oberfläche und/oder durch Behandlung mit starken Basen oder Säuren erzeugt werden (siehe z.B. die deutsche Patentanmeldung 197 20 370.1 (O.Z. 5192)). Aminogruppen lassen sich beispielsweise durch Behandlung mit einem Ammoniak-Plasma einführen.

Schließlich kann man auch nicht-funktionelle Polymere mit einem Primer versehen, der funktionelle Gruppen zur Bindung des Immunoadjuvans trägt und über diese Gruppen das Immunoadjuvans mittelbar an das Polymer bindet. Solche Primer sind z.B. Makroinitiatoren mit Hydroperoxygruppen, gamma-Aminopropyltrietoxysilan, Aminogruppen enthaltende Siloxane (siehe z.B. E.P.Plueddemann, Silane Coupling Agents, Plenum Press, 1991) und Polyethylenimimin, wie Lupasol^{(R)} von BASF AG.

Als Beispiele für Basispolymere, die funktionelle Gruppen vor ihrer Herstellung her im Bulk enthalten, als nicht funktionelle Standardpolymere durch eine nachträgliche Behandlung an der Oberfläche funktionalisiert oder durch Behandlung mit einem Primer ebenfalls an der Oberfläche mit funktionellen Gruppen zur Bindung des Immunoadjuvans versehen werden können und dann als Polymere im Sinne der Erfindung dienen können, seien genannt: Polyolefine, wie Polyethylen, Polypropylen, Polyisopren und Polyisobutylen; Polyamide, wie Polyamid-6, -11 oder -12 und Polyamid-6,6 oder -6,12; Polyester, wie Polyethylenterephthalat und Polybutylenterephthalat; Polyesteramide oder -imide, Polyetheresteramide, Polystyrol , Polyvinylchlorid, Polyacrylnitril, Polycarbonate, Polysulfone, Polysiloxane (Silicone), Polychloropren, Polytetrafluorethylen, Polyurethane, Alkyd-, Harnstoff- oder Melaminharze, Polylactide; Polyalkylenoxide, wie Polyethylenoxid und Polypropylenoxid; und Polyacrylsäure sowie entsprechende Blends oder Copolymere, Bevorzugte Polymere sind Polyurethane, Polyamide, Polyvinylchlorid und Silicone.

### 4.2 Immunoadjuvantien

Als Immunoadjuvantien, die auf den Basispolymeren fixiert werden, eignen sich die Stoffe, die allgemein als Immunoadjuvantien geeignet sind, unabhängig davon, ob es sich um natürliche oder synthetische Stoffe handelt. Es ist zu erwarten, daß sich aus vielen der für Interaktionen mit dem menschlichen oder tierischen Körper relevanten Organismen, wie Bakterien, Hefen, Pilzen, Protozoen und Viren, Präparate mit Adjuvanswirkung herstellen lassen und daß deren wirksame Prinzipien, gegebenenfalls mit strukturellen Variationen, synthetisch nachgestellt werden können. Im Prinzip sind auch Immunoadjuvantien verwendbar, die einem natürlichen Vorbild nur eingeschränkt oder gar nicht folgen. Bei der Auswahl der Immunoadjuvantien sollte darauf geachtet werden, daß sie keine oder nur geringe toxischen Nebenwirkungen haben, es sei denn, daß diese Nebenwirkungen im Rahmen einer speziellen Therapie ausdrücklich erwünscht sind, beispielsweise die Auslösung von Fieber bei speziellen Formen der Tumorbekämpfung.

Im einzelnen seien z.B. die zuvor unter der vorstehenden Ziffer 1 genannten Immunoadjuvantien erwähnt. Das von Hoechst Marion Roussel unter der Bezeichnung RU 41740 erhältliche Biostim ist ein Glycoprotein-Komplex mit unterschiedlichen funktionellen Gruppen (u.a. Hydroxyl-, Carboxyl- und Aminogruppen), der daher auf unterschiedliche Weise an Polymeroberflächen gebunden werden kann.

Die Fixierung des Immunoadjuvans auf der Polymeroberfläche erfolgt bei den bevorzugten erfindungsgemäßen Polymeren durch ionische oder kovalente Bindung, und zwar unmittelbar zwischen entsprechenden funktionellen Gruppen oder mittelbar mit Hilfe von bifunktionellen Brückenmolekülen. Eine unmittelbare ionische Bindung bildet sich z.B. aus, wenn der eine Partner Carboxylgruppen und der andere Aminogruppen enthält. In diesem Fall kann man einfach das Immunoadjuvans in wäßrigem Milieu mit dem Basispolymer in Kontakt bringen. Kovalente Bindungen können z.B. geschaffen werden, wenn man auf dem Basispolymer vorhandene Carboxylgruppen durch Behandlung mit einem anorganischen Säurechlorid in die Carbonsäurechloridgruppe umwandelt, die mit Hydroxyl- oder Aminogruppen im Molekül des Immunoadjuvans unten Ausbildung von Carbonester- oder Carbonamidgruppen reagiert. Ionische Verknüpfungen über ein Brückenmolekül sind z.B. mittels einer Dicarbonsäure möglich, wenn das Basispolymer und das Immunoadjuvans jeweils Aminogruppen enthalten. Weisen beide Partner Carboxylgruppen auf, so gelingt die ionische Verknüpfung mit Hilfe eines Diamins. Kovalente Verknüpfungen über ein Brückenmolekül können z.B. geschaffen werden, wenn sowohl das Immunoadjuvans als auch das Basispolymer Hydroxyl und/oder Aminogruppen enthalten. Als Brückenmolekül eignen sich dann z.B. Dicarbonsäuredichloride, wie Adipinsäuredichlorid, oder vorzugsweise Diisocyanate, wie Hexamethylendiisocyanat, Isophorondiisocyanat, Toluylendiisocyanat, Diphenylmethandiisocyanat und Dicyclohexylmethandiisocyanat. Da Isocyanatgruppen hydrolyseempfindlich sind, muß man das Immunoadjuvans in einem inerten Lösemittel über das Diisocyanat mit dem Basispolymer verknüpfen. In wäßrigem Milieu kann man arbeiten, wenn man ein verkapptes Diisocyanat verwendet, beispielsweise ein mit epsilon-Caprolactam oder Methylethylketoxim verkapptes Diisocyanat. In diesem Fall kann man das Immunoadjuvans zusammen mit dem verkappten Diisocyanat in wäßriger Lösung oder Suspension auf das Basispolymer aufbringen, die aufgebrachte Beschichtung trocknen, durch Erhitzen die verkappten Isocyanatgruppen entschützen und das Immunoadjuvans über Urethan- und/oder Ureidobrükken mit dem Basispolymer verknüpfen.

Die ionische oder kovalente Anbindung des Immunoadjuvans an die Polymeroberfläche mittels geeigneter funktioneller Gruppen erfolgt nach bekannten Methoden der organischen Chemie und ist für sich kein Teil der vorliegenden Erfindung.

Die Auswahl des Immunoadjuvans erfolgt in erster Linie im Hinblick auf die beabsichtigte Verwendung des erfindungsgemäßen Polymers mit auf der Oberfläche fixiertem Immunoadjuvans. Das Polymer wird u.a. danach ausgewählt, ob seine Funktionalität eine feste, vorzugsweise ionische oder kovalente Bindung des jeweiligen Immunoadjuvans gestattet. Eine solche Bindung liegt dann vor, wenn das Immunoadjuvans durch Extraktion mit Wasser oder einem anderen Lösemittel nicht oder praktisch nicht vom Polymer ablösbar ist. Eine geeignete Kombination von Polymer und Adjuvans für einen bestimmten Verwendungszweck läßt sich durch orientierende Versuche unschwer ermitteln.

### 5. Verwendung der erfindungsgemäßen Polymeren

Die neuen Polymere mit auf der Oberfläche fixierten Immunoadjuvantien eignen sich als Bakterien oder andere unerwünschte Mikroorganismen und Krankheitserreger fernhaltende Materialien. Von dieser Wirkung betroffene Bakterien sind u.a. Staphylococcus aureus, Staphylococcus epidermidis, Streptococcus pyogenes, Klebsiella pneumoniae, Pseudomonas aeruginosa und Escherichia coli . Andere betroffene schädliche Mikroorganismen sind Viren und Hefen, aber auch Protozoen, wie Trypanosoma cruzi (Infection and Immunity, **1984**, 531-535). Demzufolge eignen sich die neuen Polymere für die Herstellung von Geräten. Apparaten, Vorrichtungen und anderen Erzeugnissen, oder Teilen davon, bei deren bestimmungsgemäßer Verwendung es erforderlich oder wünschenswert ist, daß sie frei oder möglichst frei von Bakterien oder anderen schädlichen Mikroorganismen sind und bleiben. Solche Erfordernisse bestehen insbesondere auf den Gebieten der Medizin und Veterinärmedizin, der Biotechnik und der Hygienevorsorge. Im einzelnen seien Schläuche, Sonden, Katheter, Drainagen, Implantate, Wundverbände und Blutbeutel beispielhaft genannt.

Die neuen Polymere eignen sich weiter für die Herstellung von Erzeugnissen, die bei bestimmungsgemäßer Verwendung mit Tumorzellen in Berührung kommen bzw. in Nachbarschaft zu Tumorzellen gelangen. Das sind z.B. Folien für das Einsatzgebiet Tumorbekämpfung.

## Patentansprüche

1. Polymere, die auf ihrer Oberfläche fixierte Immunuadjuvantien tragen.

2. Polymere nach Anspruch 1, dadurch gekennzeichnet, daß das Basispolymer und das Immunoadjuvans durch ionische oder kovalente Bindungen miteinander verknüpft sind.

3. Polymere nach Anspruch 2, dadurch gekennzeichnet, daß das Immunoadjuvans über ein Brückenmolekül mit dem Basispolymer verknüpft ist.

4. Polymere nach einem der Ansprüche 1 bis 3, dadurch gekennzeichnet, daß das Basispolymer mit einem Primer versehen ist, an den das Immunoadjuvans gebunden ist.

5. Polymere nach einem der Ansprüche 1 bis 3, dadurch gekennzeichnet, daß das Basispolymer ein Polyurethan, Polyamid, Polyvinylchlorid oder Silicon ist.

6. Verfahren zur Herstellung der Polymeren nach den Ansprüchen 1 bis 4, dadurch gekennzeichnet, daß man ein Basispolymer und ein Immunoadjuvans in an sich bekannter Weise über geeignete funktionelle Gruppen ionisch oder kovalent miteinander verknüpft.

7. Verfahren nach Anspruch 6, dadurch gekennzeichnet, daß das Basispolymer ein polymerer Kunststoff ist, der die funktionellen Gruppen von seiner Herstellung her enthält.

8. Verfahren nach Anspruch 6, dadurch gekennzeichnet, daß das Basispolymer ein polymerer Kunststoff ist, auf dem die funktionellen Gruppen nachträglich durch eine Oberflächenbehandlung erzeugt wurden.

9. Verfahren nach einem der Ansprüche 6 bis 8, dadurch gekennzeichnet, daß das Basispolymer über einen Primer mit dem immunoadjuvans verknüpft ist.

10. Verfahren nach einem der Ansprüche 6 bis 9, dadurch gekennzeichnet, daß das gegebenenfalls mit einem Primer versehene Basispolymer und das Immunoadjuvans unmittelbar durch funktionelle Gruppen miteinander verknüpft werden.

11. Verfahren nach einem der Ansprüche 6 bis 9, dadurch gekennzeichnet, daß das gegebenenfalls mit einem Primer versehene Basispolymer und das Immunoadjuvans mittelbar über ein Brückenglied miteinander verknüpft werden.

12. Verfahren zur Herstellung von Erzeugnissen oder Teilen von Erzeugnissen, bei deren bestimmungsgemäßer Verwendung es erforderlich oder wünschenswert ist, daß sie frei oder möglichst frei von Bakterien oder anderen schädlichen Mikroorganismen sind und bleiben, dadurch gekennzeichnet, daß man dabei Polymere nach den Ansprüchen 1 bis 5 verwendet.

13. Verfahren zur Herstellung von Erzeugnissen oder Teilen von Erzeugnisse, die bei bestimmungsgemäßer Verwendung mit Tumorzellen in Berührung kommen oder sich in Nachbarschaft zu Tumorzellen befinden, dadurch gekennzeichnet, daß man dabei Polymere nach den Ansprüchen 1 bis 5 verwendet.

14. Erzeugnisse, hergestellt nach den Ansprüchen 12 oder 13.
